# EUROPEAN PATENT APPLICATION

(11) **EP 2 314 294 A2**
(43) Date of publication of application: **27.04.2011**
(21) Application number: 10185337.2
(22) Date of filing: 04.11.2005
(51) Int. Cl.: A61K 31/404, A61K 38/20, A61K 38/21, A61P 31/00, A61P 31/04, A61P 31/12, A61P 35/00

(54) **3,3'-diindolylmethane immune activating compositions**

(30) Priority: 06.11.2004 US 983414
(62) Divisional of application: 05856934.4
(71) Applicant: The Regents of The University of California, Oakland, CA 94607-5200 (US)
(72) Inventor: Bjeldanes, Leonard F, Berkeley, CA 94720-3200 (US); Riby, Jacques E, Berkeley, CA 94720-3200 (US); Xue, Ling, Berkeley, CA 94720-3200 (US); Firestone, Gary L, Berkeley, CA 94720-3200 (US)
(74) Representative: Rogers, Alex Lee

(57) **Abstract**

The invention provides immune response activating compositions and methods of use. The general methods deliver an immune response activator to a patient determined to be in need thereof, comprising the steps of: (a) administering to the patient a predetermined amount of an immune response activating, optionally substituted DIM; and (b) detecting in the patient a resultant immune response activation, such as an increase in T-cell proliferation, NO production, cytokine production, cytokine receptor expression, or cytokine signaling.

## Description

This work was supported by National Institute of Health Grant CA69056, and US Army Grant RP950844. The U.S. government may have rights in any patent issuing on this application.

### INTRODUCTION

### Field of the Invention

The field of the invention is 3,3'-diindolylmethane compositions and their use as immune activators.

### Background of the Invention

3,3'-Diindolylmethane (DIM) is a natural product formed during the autolytic breakdown of glucobrassicin present in food plants of the *Brassica* genus, including most commonly cabbage, Brussels sprouts, cauliflower and broccoli. DIM also is produced following ingestion of indole-3-carbinol (I3C), the immediate precursor of DIM in the plants (1). In addition, DIM is slowly produced from I3C under near neutral pH cell culture conditions during extended incubation periods.

Results of several studies indicate that DIM exhibits promising cancer protective activities, especially against mammary neoplasia (2-4). Oral intubation ofI3C in a single dose prior to carcinogen treatment reduced the incidence and multiplicity of DMBA-induced mammary tumors in rats by 70-80% (2,5). Repeated oral administrations of DIM during the promotion stage of DMBA-induced mammary tumorigenesis inhibited tumor growth in rodents by as much as 95% (6). We observed that under conventional cell culture conditions, DIM could inhibit the proliferation of breast tumor cell lines, regardless of estrogen receptor status (7). DIM induced a G₁ cell cycle arrest and produced a strong induction of p21 cell cycle inhibitor gene expression and promoter activity in both estrogen responsive and estrogen independent breast cancer cells. The antiproliferative effects of DIM involved Sp1/Sp3 transcription factor activation of p21 as a target for cell cycle control in human breast cancer cells (8).

Interferons (IFNs) are a group of cytokines with antiviral and cytostatic functions with an important role in the modulation of the immune response. Type I IFNs, including IFNα and IFNβ, are produced by virus-infected cells. Type II IFNs, usually called interferon-γ (IFNγ) or the "immune interferon," promote B cell differentiation into immunoglobulin-producing cells (9). The recently recognized anti-tumor activity of IFNγ is still not fully understood but it includes the priming of macrophages for non-specific tumoricidal activity, the activation of monocytes, natural killer cells and T cells to increase cytotoxicity against tumor cells, and the inhibition of tumor induced angiogenesis (10). The possible therapeutic use of IFNγ in cancer patients has been limited due to serious side effects (11).

### Relevant Literature

Chatterji, U., Riby, J.E., Taniguchi, T., Bjeldanes, E.L., Bjeldanes, L.F., and Firestone, G.L. Indole-3-carbinol stimulates transcription of the interferon gamma receptor 1 gene and augments interferon responsiveness in human breast cancer cells. Carcinogenesis, 25:1119-28, 2004.
Exon JH, South EH, "Dietary indole-3-carbinol alters immune functions in rats." J Toxicol Environ Health A. 2000 Feb 25;59(4):271-9.
J. H. Exon, E. H. South, B. A. Magnuson, K. Hendrix, "Effects of Indole-3-Carbinol on Immune Responses, Aberrant Crypt Foci, and Colonic Crypt Cell Proliferation in Rats" J Toxicol Environ Health A. 2001; 62:561-573.
Optionally substituted DIM compositions are described in copending USSN 10/664,991, filed Sep 16, 2003.
Auborn KJ, Qi M, Yan XJ, Teichberg S, Chen D, Madaio MP, Chiorazzi N. Lifespan is prolonged in autoimmune-prone (NZB/NZW) F1 mice fed a diet supplemented with indole-3-carbinol. J Nutr. 2003 Nov;133(11):3610-3.
Kohut ML, Thompson JR, Campbell J, Brown GA, Vukovich MD, Jackson DA, King DS. Ingestion of a dietary supplement containing dehydroepiandrosterone (DHEA) and androstenedione has minimal effect on immune function in middle-aged men. J Am Coll Nutr. 2003 Oct;22(5):363-71.
Stanley M. Chapter 17: Genital human papillomavirus infections--current and prospective therapies. J Natl Cancer Inst Monogr. 2003(31):117-24. Review.
Wiatrak BJ. Overview of recurrent respiratory papillomatosis. Curr Opin Otolaryngol Head Neck Surg. 2003 Dec;11(6):433-41. Review.
Auborn KJ. Therapy for recurrent respiratory papillomatosis. Antivir Ther. 2002 Mar;7(1):1-9. Review.

### SUMMARY OF THE INVENTION.

The invention provides immune response activating compositions and methods of use. The general methods deliver an immune response activator to a patient determined to be in need thereof, comprising the steps of: (a) administering to the patient a predetermined amount of an immune response activating, optionally substituted DIM; and (b) detecting a resultant immune response activation in the patient.

In particular embodiments, the immune response activation is and is detected as an increase in T-cell proliferation, NO production, cytokine production, cytokine receptor expression, or cytokines signaling; the cytokine is selected from IL-6, G-CSF, IL-12, TNF-α and IFNγ; the administering step is performed by oral or intravenous administration; and the patient is determined to be immune-compromised, or subject to an infection or a neoplasia.

The methods generally employ an immune response activating, optionally substituted 3,3'-diindolyhnethane having formula I: 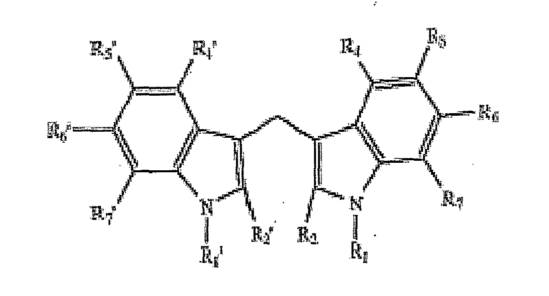 where R.sub.1, R.sub.2, R.sub.4, R.sub.5, R.sub.6, R.sub.7, R.sub.1', R.sub.2', R.sub.4', R.sub.5', R.sub.6' and R.sub.7' individually and independently, are hydrogen or a substituent selected from the group consisting of a halogen, a hydroxyl, a linear or branched alkyl or alkoxy group of one to ten carbons, an amine, a sulfonyl, and a nitro group. In particular embodiments, the compound includes at least one such substituent, preferably at a position other than, or in addition to R.sub 1 and R.sub 1', the linear or branched alkyl or alkoxy group is one to five carbons, and/or the halogen is selected from the group consisting of chlorine, bromine and fluorine. In particular embodiments, the indolyls are symmetrically substituted, wherein each indolyl is similarly mono-, di-, tri-, or para- substituted.

The invention also provides methods of using an immune response activating, optionally substituted 3,3'-diindolylmethane in conjunction with one or more other therapeutic agents, particularly different immune response activating or anti-infection or anti-neoplasia compounds, for complementary, additive, and/or synergistic efficacy. These methods may employ combination compositions, which may be in combination unit dosages, or separate compositions, which may be provided separately dosed in joint packaging.

The invention also provides kits comprising an immune response activating, optionally substituted 3,3'-diindolylmethane, and an instructional medium reciting a subject method. The recited immune response activating, optionally substituted 3,3'-diindolylmethane may be present in premeasured, unit dosage, and may be combined in dosage or packaging with an additional therapeutic agent, particularly a different immune response activator, or an anti-infection or anti-neoplasia compound.

### DETAILED DESCRIPTION OF PARTICULAR EMBODIMENTS OF THE INVENTION

The following descriptions of particular embodiments and examples are offered by way of illustration and not by way of limitation. Unless contraindicated or noted otherwise, in these descriptions and throughout this specification, the terms "a" and "an" mean one or more, the term "or" means and/or.

The general methods deliver an immune response activator to a host determined to be in need thereof, comprising the steps of: (a) administering to the patient a predetermined amount of an immune response activating, optionally substituted 3,3'-diindolylmethane (DIM); and (b) detecting a resultant immune response activation in the patient. The method may further comprise, prior to the contacting step, determining that the host is in need of the immune response activator.

The disclosed compositions provide diverse applications to hosts determined to be in need of an immune response activator, and in particular embodiments, determined to be in need of an increase in T-cell proliferation, NO production, cytokine production, cytokine receptor expression, or cytokine signaling, particularly wherein the cytokine is selected from IL-6, G-CSF, IL-12, TNF-α and IFNγ. For example, scientists can use the compositions to provide immune response activating activity to cell cultures or laboratory animals. Preferred hosts are human patients, wherein the compositions may be administered to patients determined to be subject or predisposed to any of the wide variety of disorders known to be treatable with the subject immune response activators. Examples include use as an anti-infection therapy in viral, bacterial and fungal infections.

In a preferred embodiment, the host is a human patient determined to be subject or predisposed to a pathology which can be ameliorated with an increased immune response, and wherein the resultant immune response activation of the invention is manifested as a reduction in the pathology or progress of the pathology, particularly a pathology selected from the group consisting of viral, bacterial and fungal infections.

Our methods generally employ an immune response activating, optionally substituted 3,3'-diindolylmethane having the structure of formula I, wherein R.sub.1, R.sub.2, R.sub.4, R.sub.5, R.sub.6, R.sub.7, R.sub.1', R.sub.2', R.sub.4', R.sub.5', R.sub.6' and R.sub.7' individually and independently, are hydrogen or a substituent selected from the group consisting of a halogen, a hydroxyl, a linear or branched alkyl or alkoxy group of one to ten carbons, an amine, a sulfonyl, and a nitro group. Substituent-containing compounds may be referred to as DIM derivatives or DIM analogs. In particular embodiments, the compound includes at least one such substituent, preferably at a position other than, or in addition to R.sub 1 and R.sub 1', the linear or branched alkyl or alkoxy group is one to five carbons, and/or the halogen is selected from the group consisting of chlorine, bromine and fluorine.

Immune response activating activity is readily confirmed with the various assays described below, including cytokine and cytokine receptor assays, T-cell proliferation assays, NO production asssay, etc., which may be practiced in cell culture and in a wide variety of clinically relevant and validated animal models.

In particular, we devised an iterative, combinatorial synthetic scheme to generate a library of DIM derivatives for high-throughput screening of immune response activating activity. In an exemplary demonstration, we generated 451 DIM derivative structures in five synthetic rounds, summarized below:
- Synthetic round 2:: R sub.2, 4, 5, 6 or 7, R sub.2', 4', 5', 6' or 7' di-F, -Cl, or -Br-3,3'-diindolylmethane
- Synthetic round 5:: R sub.2, 4, 5, 6 or 7, R sub.2', 4', 5', 6' or 7' di-methyl-, ethyl-, propyl-, butyl-, or pentyl-3,3'-diindolylmethane
- Synthetic round 6:: R sub.2, 4, 5, 6 or 7, R sub.2', 4', 5', 6' or 7' di-methoxy-, ethoxy-, Synthetic round 9: propyloxy-, butyloxy-, or pentyloxy-3,3'-diindolylmethane R sub.2, 4, 5, 6 or 7, R sub.2', 4', 5', 6' or 7' di-hydroxyl, amino-, aminomethyl-, sulfo-, or nitro-3,3'-diindolylmethane
- Synthetic round 12:: R sub.2, 4, 5, 6, 7, R sub. 2', 4', 5', 6', 7' deca-fluoro. (perfluoro)-3,3'-diindolylmethane

Immune response activating effects of the DIM analogs are examined with cell-based assays for cytokine and cytokine receptor expression. In our initial demonstrations, we used a cDNA gene expression microarray screen of treated and untreated human breast cancer MCF-7 cells, followed by confirmation of results using Northern blots and quantitative RT-PCR, to monitor transcriptional activation of IFNγ, IFNγ receptor (IFNGR1), oligoadenylate-synthase (OAS) family member protein 69 (p69-OAS), and interferon-inducible protein 56 (p56), a protein closely related to OAS. We also used a sensitive sandwich ELISA assay to examine levels of IFNγ in medium from confluent cultures of MCF-7 cells that were treated for up to 3 days. DIM derivatives confirmed to have immune response activating activity in our reporter and ELISA assays are subsequently screened in a whole animal cytokine production and Y. enterocolitica infection studies, as described below in Examples VIII and IX.

**Table 1. Immune response activating substituted 3,3'-diindolylmethane compounds**

| | |
|---|---|
| 5,5'- dichloro-diindolylmethane | 5,5'-dibromo-diindolylmethane |
| 5,5'-difluoro-diindolylmethane | perfluoro-3,3'-diindolylmethane |
| 5,5'-dimethyl-diindolylmethane | 5,5'-diethyl-diindolylmethane |
| 5,5'-dipropyl-diindolylmethane | 5,5'-dibutyl-diindolylmethane |
| 5,5'-dipentyl-diindolylmethane | 5,5'-dimethoxy-diindolylmethane |
| 5,5'-diethoxy-diindolylmethane | 5,5'-dipropyloxy-diindolylmethane |
| 5,5'-dibutyloxy-diindolylmethane | 5,5'-diamyloxy- diindolylmethane |
| N,N'- dimethyl-diindolylmethane | N,N'-diethyl-diindolylmethane |
| N,N'-dipropyl-diindolylmethane | N,N`-dibutyl-diindolylmethane |
| N,N'-dipentyl-diindolylmethane | 2,2'-dimethyl-diindolylmethane |
| 2,2'-diethyl-diindolylmethane | 2,2'-dipropyl-diindolylmethane |
| 2,2'-dibutyl- diindolylmethane | 2,2'-dipentyl-diindolylmethane |

In particular embodiments, the indolyl moieties are symmetrically substituted, wherein each moiety is similarly mono-, di-, tri-, etc. substituted. In other particular embodiments, R.sub.1, R.sub.2, R.sub.4, R.sub.6, R.sub.7, R.sub.1', R.sub.2', R.sub.4', R.sub.6', and R.sub.7' are hydrogen, and R.sub.5 and R.sub.5' are a halogen selected from the group consisting of chlorine, bromine and fluorine. Additional DIM derivatives from which immune response activating compounds are identified as described herein include compounds wherein R.sub.1, R.sub.2, R.sub.4, R.sub.6, R.sub.7, R.sub.1', R.sub.2', R.sub.4', R.sub.6', and R.sub.7' are hydrogen, and R.sub.5 and R.sub.5' are halogen. These include, but are not limited to 3,3'-diindolylmethane, 5,5'- dichloro-diindolylmethane; 5,5'-dibromo-diindolylmethane; and 5,5'-difluoro-diindolylmethane. Additional preferred such DIM derivatives include compounds wherein R.sub.1, R.sub.2, R.sub.4, R.sub.6, R.sub.7, R.sub.1', R.sub.2', R.sub.4', R.sub.6', and R.sub.7' are hydrogen, and R.sub.5 and R.sub.5' are an alkyl or alkoxyl having from one to ten carbons, and most preferably one to five carbons. These include, but are not limited to 5,5'-dimethyl-diindolylmethane, 5,5'-diethyl-diindolylmethane, 5,5'-dipropyl- diindolylmethane, 5,5'-dibutyl-diindolylmethane and 5,5'-dipentyl-diindolylmethane. These also include, but are not limited to, 5,5'-dimethoxy-diindolylmethane, 5,5'-diethoxy-diindolylmethane, 5,5'-dipropyloxy-diindolylmethane, 5,5'-dibutyloxy-diindolylmethane, and 5,5'-diamyloxy-diindolylmethane.

Additional preferred such DIM derivatives include compounds wherein R.sub.2, R.sub.4, R.sub.5, R.sub.6, R.sub.7, R.sub.2', R.sub.4', R.sub.5', R.sub.6', and R.sub.7' are hydrogen, and R.sub.1 and R.sub.1' are an alkyl or alkoxyl having from one to ten carbons, and most preferably one to five carbons. Such useful derivatives include, but are not limited to, N,N'- dimethyl-diindolylmethane, N,N'-diethyl-diindolylmethane, N,N'-dipropyl-diindolylmethane, N,N'-dibutyl-diindolylmethane, and N,N'-dipentyl-diindolylmethane. In yet another preferred embodiment, R.sub.1, R.sub.4, R.sub.5, R.sub.6, R.sub.7, R.sub.1','R.sub.4', R.sub.5', R.sub.6', and R.sub.7' are hydrogen, and R.sub.2 and R.sub.2' are alkyl of one to ten carbons, and most preferably one to five carbons. Such compounds include, but are not limited to, 2,2'-dimethyl- diindolylmethane, 2,2'-diethyl-diindolylmethane, 2,2'-dipropyl-diindolylmethane, 2,2'-dibutyl-diindolylmethane, and 2,2'-dipentyl-diindolylmethane. In another embodiment, R.sub.1, R.sub.2, R.sub.4, R.sub.6, R.sub.7, Rub.1 R.sub.2', R.sub.4', R.sub.6', and R.sub.7' are hydrogen, and R.sub.5 and R.sub.5' are nitro.

Substituted DIM analogs are readily prepared by condensation of formaldehyde with commercially available substituted indoles. Precursor compounds can be synthesized by dimethylformamide condensation of a suitable substituted indole to form a substituted indole-3-aldehyde. Suitable substituted indoles include indoles having substituents at R.sub.1, R.sub.2, R.sub.4, R.sub.5, R.sub.6 and R.sub.7 positions. These include, but are not limited to 5-methoxy, 5-chloro, 5-bromo, 5-fluoro, 5'-methyl, 5-nitro, n-methyl and 2- methyl indoles. The substituted indole 3-aldehyde product is treated with a suitable alcohol such as methanol and solid sodium borohydride to reduce the aldehyde moiety to give substituted I3Cs. Substituted DIMs are prepared by condensing the substituted indole-3-carbinol products. This may be achieved, for example, by treatment with a phosphate buffer having a pH of around 5.5.

The subject compositions may be administered along with a pharmaceutical carrier and/or diluent. Examples of pharmaceutical carriers or diluents useful in the present invention include any physiological buffered medium, i.e., about pH 7.0 to 7.4 comprising a suitable water soluble organic carrier. Suitable water-soluble organic carriers include, but are not limited to corn oil, dimethylsulfoxide, gelatin capsules, etc. The immune response activating compositions of the present invention may also be administered in combination with other agents, for example, in association with other chemotherapeutic or immunostimulating drugs or therapeutic agents. These methods may employ combination compositions, which may be in combination unit dosages, or separate compositions, which may be provided separately dosed in joint packaging.

In particular embodiments, the invention provides methods of using the subject immune response activating, optionally substituted 3,3'-diindolylmethane in conjunction with one or more other therapeutic agents, particularly different immune response activating compounds, for complementary, additive, and/or synergistic efficacy. Cytokines provide exemplary, well-known immune response activators useful in such combinations, and include epidermal growth factor (EGF), platelet-derived growth factor (PDGF), fibroblast growth factors (FGFs), transforming growth factors-β (TGFs-β), transforming growth factor-α (TGF-α), erythropoietin (Epo), insulin-like growth factor-I (IGF-I), insulin-like growth factor-II (IGF-II), interleukin-1 (IL-1), interleukin-2 (IL-2), interleukin-6 (IL-6), interleukin-8 (IL-8), tumor necrosis factor-α(TNF-α), tumor necrosis factor-β (TNF-β), interferon-γ (INF-γ), colony stimulating factors (CSFs), etc. Additional cytokines suitable for coadministration in the subject methods are described in Cancer Medicine. 6th ed. Kufe, et al., editors, Hamilton (Canada): BC Decker Inc; c2003), and in The Cytokine Handbook, Fourth Edition, Two Volume Set, Academic Press; 4 edition (Thomson et al., July 7, 2003).

The compositions of the present invention may be administered orally, intravenously, intranasally, rectally, or by any means which delivers an effective amount of the active agent(s) to the tissue or site to be treated. Suitable dosages are those which achieve the desired endpoint. It will be appreciated that different dosages may be required for treating different disorders. An effective amount of an agent is that amount which causes a significant decrease in the targeted pathology, or progress of the pathology, or which delays the onset or reduces the likelihood of pathology in predisposed hosts. For example, the effective amount may decrease infectious agent count, growth rate, associated pathology, etc.

The administered immune response activating, optionally substituted DIM may be advantageously complexed or coadministered with one or more functional moiety to provide enhanced update, bioavailability, stability, half-life, etc., or to reduce toxicity, etc. However, the compositions nevertheless comprise the recited immune response activating, optionally substituted DIM, whether in isolated, complexed, or a pro-drug form.

Those having ordinary skill in the art will be able to empirically ascertain the most effective dose and times for administering the agents of the present invention, considering route of delivery, metabolism of the compound, and other pharmacokinetic parameters such as volume of distribution, clearance, age of the subject, etc.

The invention also provides kits specifically tailored to practicing the subject methods, including kits comprising an immune response activating, optionally substituted 3,3'-diindolylmethane, and an associated, such as copackaged, instructional medium describing or reciting a subject method. The recited immune response activating, optionally substituted 3,3'-diindolylmethane may be present in premeasured, unit dosage, and may be combined in dosage or packaging with an additional therapeutic agent, particularly a different immune response activator, particularly a cytokine.

The invention also provides business methods specifically tailored to practicing the subject methods. For example, in one embodiment, the business methods comprise selling, contracting, or licensing a subject, immune response activating, optionally substituted 3,3'-diindolylmethane-based method or composition.

The present invention is exemplified in terms of in vitro and in vivo activity against infection in various neoplastic and normal cell lines. The test cell lines employed in the in vitro assays are well recognized and accepted as models for immune response activation in animals. The mouse experimental in vivo assays are also well recognized and accepted as predictive of in vivo activity in other animals such as, but not limited to, humans.

### EXEMPLARY EMPIRICAL PROTOCOLS

In protocols I - VII, we show that DIM activates the IFNγ signal transduction pathway. Human breast cancer MCF-7 cells expressed IFNγ mRNA and secreted IFNγ protein into their culture medium, in response to DIM treatment. DIM treatment also activated the expression of IFNGR1 and the IFNγ responsive genes, p56 and p69-OAS. DIM also produced a synergistic activation with IFNγ of interferon-inducible-reporter constructs in transfected cells. In addition, DIM also augmented the phosphorylation of STAT-1, providing further evidence of DIM activation of the IFNγ pathway. DIM and IFNγ exhibited additive antiproliferative activities in cultured cells. Finally, combined treatments with DIM and IFNγ produced synergistic increases in expression of major histocompatability complex class I (MHC-I), consistent with a strong immune potentiation activity of DIM.

### I. DIM activates transcription of IFNγ and related genes.

An initial cDNA gene expression microarray screen of human breast cancer MCF-7 cells treated with DIM, followed by confirmation of results using Northern blots and quantitative RT-PCR, established the transcriptional activation of IFNγ, IFNγ receptor (IFNGR1), oligoadenylate-synthase (OAS) family member protein 69 (p69-OAS), and interferon-inducible protein 56 (p56), a protein closely related to OAS. The expressions of IFNγ, p69-OAS and p56 were fully induced following 6 h of treatment, whereas maximum IFNGR-1 induction was reached following 48 h of treatment. Levels of mRNA for IFNγ and p56 returned to near control levels following 48 h of DIM treatment. mRNA levels for IFNGR1 and p69-OAS remained elevated at 48 h of treatment. Maximum induction for IFNγ, IFNGR1, p69-OAS and p56 were 4.6, 3.8, 3.2 and 4.0 fold, respectively.

### II. DIM induces expression of IFNγ and IFNGR1 proteins.

Following the observations that levels of mRNA for IFNγ and IFNGR1 were up-regulated by DIM, we examined levels of the protein products of these genes. Using a sensitive sandwich ELISA assay, we examined levels of IFNγ in medium from confluent cultures of MCF-7 cells that were treated with DIM for up to 3 days. The results of these studies showed a gradual accumulation of IFNγ in the medium of untreated cells and a more rapid increase in secretion of IFNγ from cells incubated with DIM. Levels of IFNγ in the medium of cultured cells were increased following incubations for 1 and 3 days from virtually undetectable levels in the vehicle treated control to approximately 50 ± 10 pg/mL and 80 ± 30 pg/mL, respectively, for the indole treated cells.

Results of Western blot analysis showed that levels of IFNGR1 protein were strongly up-regulated by DIM treatments. IFNGR1 protein levels increased by approximately 7 fold after 24 h and persisted to 48 h compared to DMSO-treated controls. These results are consistent with the increase in mRNA levels and indicate that DIM can regulate IFNGR1 expression through transcriptional control.

### III. DIM augments IFNγ-induced expression of the endogenous p69-OAS gene and associated reporter gene constructs.

We next examined in more detail the effects of DIM alone and in combination with IFNγ on expression of endogenous p69-OAS and on expression of transfected IFNγ-responsive reporter gene constructs.

The results of RT-PCR analyses of p69-OAS transcripts showed that separate treatments with DIM (50 µM) and IFNγ (10 ng/mL) produced inductions of approximately 3.5- and 5.0-fold, respectively, over the control, whereas treatment with a combination of the two produced an increase of nearly 12-fold. These results indicate a synergistic interaction of DIM and IFNγ on induced expression of endogenous p69-OAS.

We determined next whether the inducing effects of DIM on endogenous p69-OAS gene expression required concurrent protein synthesis. Our results indicate that co-treatments with the translation inhibitor, cycloheximide, blocked the inducing effects of DIM treatments, as well as, the enhancing effects of DIM on IFNγ-induced expression of endogenous p69-OAS.

In a further experiment to examine whether the inducing effects of DIM are at the transcriptional level, the effect of DIM on activities of two luciferase reporter constructs, OAS-Luc (12) and 4GAS-Luc (13) was examined. OAS-Luc contains the promoter and 5'-flanking region (-972) of p69-OAS and GAS-Luc contains four repeats of the consensus GAS element. MCF-7 cells transiently transfected with these reporter constructs were treated with IFNγ, DIM, or a combination of both for 24 h. DIM treatment induced expression of the OAS-Luc reporter to a maximum of about 2.3 fold, a level of induction that was somewhat less than the level we observed for induction of the corresponding endogenous gene of over 3-fold. IFNγ induced transcriptional activity of this reporter in a concentration dependent manner to a maximum of about 1.8 fold, which is less than the response of the endogenous gene of about 5.0 fold. In marked contrast to the maximum effects of combined treatments of the inducers on endogenous gene expression (nearly 12-fold increase compared to vehicle treated cells), the reporter gene construct responded with less than a 3-fold increase over the controls. These results show that whereas the reporter and endogenous gene responded similarly to treatment with DIM by itself, the reporter was less responsive than the endogenous gene to IFNγ.

Finally, studies with cells transfected with the 4GAS-Luc reporter showed an inducing effect of DIM that was similar to the responses of the p69-OAS gene, but a more robust response to IFNγ that was accompanied by a consistent synergistic response to co-treatments with the two inducers. Thus, treatment with DIM (50 µM) by itself again produced a 2 to 3-fold increase in activity of this reporter. IFNγ treatments, however, produced a robust concentration dependent increase in reporter activity to approximately 40-fold induction compared to vehicle treated controls. Co-treatments with DIM and IFNγ produced a roughly 2-fold synergistic activation of the 4GAS-Luc reporter at all IFN-γ concentrations examined, reaching a maximum induction of approximately 75 fold.

Taken together, these results show that DIM by itself produced similar levels of activation of endogenous and transfected reporter genes that are differentially responsive to IFNγ. The results show further that the inducing effects of DIM are mediated by a short lived transcription factor or require de novo synthesis of an intermediate regulatory protein.

### IV. Synergistic effects of DIM on IFNγ-mediated signal transduction.

To acsertain the role of GAS element activation in transcriptional activation by DIM, we examined the effect of this indole on downstream events in the GAS signaling pathway. For this purpose, the effects of DIM on phosphorylation of the signal transduction and activator of transcription factor 1 (STAT1), and the binding of the activated STAT1 dimer to the cognate GAS responsive element were examined. Phosphorylation of STAT1 by Janus-activated kinase (JAK) is the first step in IFNγ signaling following binding to IFNGR1. For these studies, cells were pretreated with DIM for 6 or 24 h followed by a 15 min. exposure to IFNγ. STAT1 phosphorylation levels in cell lysates of DIM-treated cells were compared to controls that received IFNγ but had not been treated with DIM. Phosphorylation was measured by Western blot analysis using an antibody specific to STAT1 phosphorylated on Tyr-701 residue. Our results show that whereas DIM treatment by itself for 6 or 24 h did not induce STAT1 phosphorylation, pretreatment with DIM increased STAT1 phosphorylation that was induced by IFNγ. None of the treatments affected the levels of inactive (total) STAT 1.

An electrophoretic gel mobility shift assay was used to verify that the phosphorylated STAT1 was effectively activated to a dimer that could bind to the GAS element. The ³²P-labeled DNA probe containing a GAS sequence was incubated with nuclear extracts from cells pretreated with DIM for various times as indicated, and with IFNγ for 30 min. IFNγ treatment produced a band-shift that increased in intensity with the duration of the DIM pretreatment. DIM treatments in absence of IFNγ did not produce a shifted band at any time. When samples were incubated with an antibody specific to STAT1, the band was super-shifted, confirming the identity of the protein binding to the labeled GAS probe.

Taken together, these results show that DIM can augment IFNγ-induced STAT1 activation, dimerization and binding to GAS element in DNA. The results indicate that the synergistic effects of DIM on IFNγ activity are clearly evident at several levels in IFNγ signaling and may be the most significant effect of DIM under these assay conditions.

### V. Effects of DIM and IFNγ on proliferation and cell cycling.

Functional consequences of the synergistic effects of DIM and IFNγ on gene expression were examined at the level of proliferation rates. Cell proliferation was measured over a 4-day treatment period with treatments with DIM, IFNγ, or both substances, and compared to a DMSO control. With heat-treated serum, IFNγ alone reduced cell proliferation by as much as 70% compared to controls. DIM treatments reduced proliferation by as much as 60%. With the exception of the highest concentration of IFNγ, combined treatments with DIM and IFNγ resulted in roughly additive increases in the cytostatic activity compared to treatment with DIM by itself. These effects were accompanied by morphological changes characteristic of apoptosis. We observed no significant effect of IFNγ on proliferation of cells grown in unheated serum.

Flow cytometry was used to determine the effects of DIM and IFNγ on cell cycling. Our data showed the proportion of the cell population in the G1 phase of the cell cycle at different intervals up to 3 days. In control groups approximately 50 % of the cells were in G1. Treatment with IFNγ alone caused a small progressive increase in G1 blocked cells of up to 10 % above the DMSO control, after 3 days. DIM had a more immediate effect that increased over time up to 30% above the DMSO control. The combination of DIM and IFNγ had an additive effect leading to more than 90% of the cells in G1 arrest after three days, correlating with the absence of proliferation. Taken together, these results are consistent with separate cytostatic mechanisms for DIM and IFNγ.

### VI. DIM potentiates IFNγ induced expression of MHC-I complex.

Induced expression of the MHC-I complex is a well-established and important down-stream target of IFNγ-mediated signal transduction. To examine further the possible synergistic effects of DIM on a significant metabolic product of IFNγ signaling, we tested the effects of co-treatment with DIM on IFNγ induced expression of MHC-I complex in MCF-7 cells.

Flow cytometry analysis of cell surface MHC-I expression was conducted using FITC-conjugated HLA-ABC antibody. In an initial control experiment, cultured MCF-7 cells were pre-treated with 30 µM DIM for 48 hours and then with or without 10 ng/mL IFNγ for another 16 hours. Analyses using a negative control antibody, FITC-conjugated mouse IgG2b, indicated no significantly induced signal. In a subsequent experiment in which cells were treated with a range of IFNγ concentrations and analyzed with the anti-MHC-I antibody, a strong signal indicative of IFNγ-induced MHC-I expression was detectable at 0.1 ng/mL and appeared to plateau at 10 ng/mL. In cells treated with vehicle control only, MHC-I expression was not detected in greater than 99% of cells.

Pretreatment with 30 µM DIM for 48 h followed by treatment with 0.1 ng/mL IFNγ produced a further increase in the percentage of MHC-I positive cells from 8.11% to 40.98%, but had no significant effect on MHC-I expression/cell (MFV). Exposure to DIM of cells treated with 10 ng/mL of IFNγ, about 95% of which expressed MHC-I, produced a strong time dependent increase in MFV from 76.04 to 131.41. DIM treatment alone had no significant effect on the expression of the MHC-I complex. As expected, addition of an IFNγ blocking antibody into the medium before the treatments abrogated the inducing effects of co-treatments of IFNγ and DIM on MHC-I expression, confirming the requirement of the cytokine for the observed effects.

In an analogous further experiment, we observed no effect of DIM or IFNγ on MHC-II expression in the MCF-7 cells. These results show that whereas DIM by itself produces little or no effect on expression of MHC-I, this indole strongly augments the proportion of cells that express this protein complex, as well as the maximum level of MHC-I per cell, in response to IFNγ.

### VII. DIM enhances IFNγ-induced transcription of MHC-I components and transporters.

Because the level of the MHC-I protein complex on the cell surface could be enhanced by pretreatment with DIM, we examined whether expressions of the corresponding genes for the complex and its transporters were also increased. RT-PCR was used to examine the expression of the four major components of human MHC-I , including HLA-A, HLA-B, HLA-C, and β-microtubulin, as well as two important associated transporters, TAP1 and TAP2. Our results indicate that the expression levels of all six genes were not significantly enhanced by DIM compared to vehicle treated MCF-7 cells. As expected, the expressions of these genes were increased 2-8 fold by treatment with IFNγ. The results show, in addition, that pretreatment of cells with DIM further increased the mRNA levels of these genes by at least 2 fold above the levels induced by IFNγ by itself, or to 4-16 fold above background levels.

These results show that DIM can synergistically enhance the level of IFNγ-induced expression of the MHC-I protein complex and associated mRNAs. The roughly 2-fold augmentation by DIM in levels of both MHC-I protein/cell and in the associated mRNAs, indicates that MHC-1 expression by DIM is regulated at the level of gene transcription.

The results of protocols I-VII indicate that DIM is an immunomodulator that can induce expression of IFNγ and influence the response of cells to exogenous exposure to this cytokine. The results show that DIM can 1) induce the expression of IFNγ, the IFNγ receptor, and two IFNγ-inducible genes, 2) induce the expression of IFNγ-inducible reporter gene constructs, 3) synergistically augment IFNγ-mediated activation of the STAT-1 signal transduction pathway, 4) additively augment the cytostatic effects of IFNγ in cultured cells, and 5) synergistically augment the expression of IFNγ-induced MHC-I protein complex and associated mRNAs. To our knowledge, this is the first report of production of IFNγ by tumor cells. Following treatment with DIM, we observed strong signals for secreted IFNγ using an ELISA technique.

Activation of IFN-γ expression in immune cells is mediated by a complex interaction of signaling processes. Only lymphocytes that promote innate or adaptive immunity are known to produce IFNγ. IFNγ production in these cells is normally controlled by secreted cytokines, the most extensively studied of which is interleukin (IL)-12 (14). Exposure of immune cells to IL-12 results in the activation of the Janus kinases, JAK2 and Tyk2, which in turn phosphorylate the IL-12 receptor, providing docking sites for the transcription factor STAT4. Receptor-bound STAT4 is phosphorylated by the JAKs, which promotes STAT4 dimerization, translocation to the nucleus, and regulation of gene expression. STAT4 may contribute directly to IFNγ gene regulation, since potential STAT4 binding sites are present in the first intron and promoter of the IFNγ gene (15). Our studies indicate that DIM induction of IFNγ expression in tumor cells proceeds by a direct effect on gene activation that does not involve the intermediacy of other induced cytokines. The increase in transcription occurs within only 6 hours, a period generally too short for protein synthesis; furthermore, MCF-7 cells are not known to produce IFNγ-activating cytokines such as IL-12.

The signaling cascade that is regulated by IFNγ has been examined in considerable detail. Binding of IFNγ to the highly specific IFNγ receptor (IFNGR1) on the membrane of target cells normally activates a phosphorylation cascade involving JAK1 and JAK2 and STAT1. Phosphorylated STAT1 homodimerizes, translocates to the nucleus, and binds to the IFNγ activated sequence (GAS) in the promoter of IFNγ-inducible genes, and activates transcription. Activation, in turn, of one such gene, p69-OAS, plays an important role in the prevention of replication of viral RNA in infected cells. In addition, p69-OAS has been recently identified as an inhibitor of cell growth and a pro-apoptotic protein related to Bcl-2 (17). Our results confirm that IFNγ can activate the JAK/STAT pathway in breast tumor cells, and show that this activity of IFNγ (i.e. STAT1phosphorylation, STAT1 dimerization and binding to GAS, and activation of GAS regulated transcription) is synergistically augmented by treatments with DIM. These results show that whereas DIM has limited effect by itself on immediate down-stream events of IFNGR1 activation, this indole strongly enhances the effects of added IFNγ.

The effects of DIM on IFNγ signaling are clearly distinguishable from the effects of these other substances. In one series of studies, the synergistic effects of retinoic acid (RA) and IFNγ in breast cancer cells were described. Initially, a synergistic antiproliferative effect was observed following sequential treatment of cells with IFNγ and RA. The effect appeared to result from an IFNγ-mediated augmentation of RA cytostatic activity. IFNγ was shown to increase RA cytostatic potency by increasing expression of retinoic acid receptor-gamma (RARγ) levels and decreasing expression of cellular RA-binding proteins (CRABP) (18). Subsequently, the effects of RA on IFNγ signaling were also examined in breast tumor cells. The results showed that RA could synergistically augment the effects of IFNγ on gene transcription in MCF-7 cells by a mechanism that involved RA-mediated augmentation of STAT1 expression (19). In another series of studies the effects of combined treatments of breast cancer cells with IFNs and the estrogen antagonist, tamoxifen, were examined. Co-treatments with these substances caused an augmentation of the expression of certain IFN-stimulated genes, including the transcription factors ISGF-3 and GAF (20). Tamoxifen by itself, however, produced no effect on the expression of these transcription factors. Thus, our results with DIM show significant differences from the reported effects of RA and tamoxifen on IFNγ action. We observed an additive inhibitory effect of DIM and IFNγ on MCF-7 cell proliferation, whereas the effect with RA was synergistic. Also in contrast to RA, although DIM synergistically augmented IFNγ signal transduction, this indole did not increase STAT1 expression in the absence of IFNγ treatment. Finally, DIM treatment by itself clearly induced expression of IFNGR1, IFNγ and OAS-related genes, effects that were not reported for RA or tamoxifen.

The synergistic effect of DIM on IFNγ-induced expression of MHC-I is of considerable importance since this complex plays an important role in tumor immunosurveillance. MHC-I molecules are required for the presentation of tumor-associated antigen (TAA) to cytotoxic T-lymphocytes (CTLs). Decreased expression of MHC antigen can protect tumor cells from immunosurveillance (21, 22). Accordingly, loss or down-regulation of MHC-I has been shown to be a frequent event in breast tumorigenesis. Indeed, several studies with murine models of induced carcinogenesis have confirmed the role of down-regulation of MHC-I antigens in increasing the tumorigenic and metastatic potential of tumors (23). Conversely, induced MHC-I expression is important in antitumorigenic properties of IFNγ (24) and certain small molecule cancer therapeutic agents, including the nucleotide analog, 5-azacytidine cystosine arbinoside, 5-flurouracil, retinoids, vitamin D3, as well as the plant alkaloid, vincristine (25, 26).

Comparison of our results with DIM to our studies with I3C (27) show some distinct differences in the activity of DIM and its in vivo precursor in their effects on IFNγ signaling. Similar to our current results with DIM, we showed in the previous work that I3C also can affect IFNγ signaling in MCF-7 cells by a mechanism that involves a strong and rapid increase in expression of IFNGR1 and synergistic activation of STAT1 signaling. Similar effects of DIM and I3C were also observed on cell proliferation and cell cycling. In contrast to the present results with DIM, however, we did not observe in the previous studies increased expression by I3C of the IFNγ-stimulated genes (p56 and p69-OAS). Although the maximum levels of IFNGR1 protein induction were similar for I3C and DIM, i.e. about 7 fold, the kinetics of receptor expression were significantly different following the two treatments. Thus, a strong level of induction (about 5 fold) was seen after only 6 hours of exposure to DIM, and the maximum induction was attained by 24 hours of treatment with this diindole. In contrast, receptor expression was increased by only about 3 fold following 24 hours of treatment with I3C, and appeared to reach the maximum induction only after about 48 hours of exposure. These results are consistent with the hypothesis that the effects of I3C on IFNGR1 expression are mediated by its slow conversion to DIM during incubation with the cells. Indeed, we have shown previously that DIM accumulates in the nucleus of MCF-7 cultured cells treated with I3C (28).

Our results explain the clinical effectiveness of indole treatments in the control of recurrent respiratory papillomatosis (RRP). I3C and DIM have become popular adjunct therapies for this disorder because of their effectiveness and low level of toxicity (29,30). RRP is caused by certain types of human papilloma viruses (HPVs) (31, 32), and a hallmark of this disease is the tendency of the papillomas to recur after surgical removal (33-34). One report indicated that most patients (55.4%) respond to the treatment ofI3C/DIM by slowing down the recurrence rate, and recurrence of the disease is completely inhibited in 19% of patients (30). Previously suggested modes of action ofI3C/DIM in the control of RRP include induction of a better estrogen metabolite balance (35), inhibition of cell proliferation (36), and induction of apoptosis (37). Our results indicate that I3C/DIM can function by yet another mechanism, that of immune potentiation. This activity is not only useful in the prevention of recurrent papillomas, but also in treatment of papillomatosis and the prevention of malignant conversion of a broad range of tumor types.

We observed the immune response activating activity of DIM at physiologically relevant concentrations. A man of average weight who consumes 200 g of broccoli daily will obtain approximately 12 mg of DIM. With maximum absorption of DIM, the blood concentration of DIM will reach approximately 10 µM. Therefore, *in vivo* concentrations of DIM from dietary *Brassica* vegetables represent the effective levels of DIM *in vitro.*

### VIII. 3,3'-diindolylmethane and substituted 3,3'-diindolylmethane stimulate a time-dependent increase in serum levels of cytokines.

In this study, we investigated the effects of DIM on immune response activation, including production of cytokine in vivo and in vitro, T cell proliferation, and NO production.

We firstly investigated the effect of unsubstituted DIM on the production of IL-6, G-CSF, IL-12, TNF-α and IFNγ. A groups of 3 mice was given 30 µg/kg of unsubstituted DIM orally and then sacrificed at 1, 3, 5, 8, and 24 h after drug challenge. Serum was collected, pooled, and assayed for IL-6, G-CSF, IL-12 and TNF-α by ELISA. Results show that DIM stimulated a time-dependent increase in serum levels of cytokines. DIM produced an increase in circulating levels of G-CSF and TNF- α with the 3 h time point being maximal. IL-6 was also induced with peak at 5 h. DIM also stimulated IL-12 and kinetics were slightly different since the increased levels were sustained for at least 24 h. In analogous experiments, we found elevated cytokine production, including elevated IFNγ production, in mice administered DIM intravenously.

We designed parallel subsequent studies to examine the effects of the panel of exemplary subsitituted DIM compounds shown in Table 1 (supra) on immune response activation, including production of cytokine in vivo and in vitro, T cell proliferation, and NO production. These experiments demonstrate similar time-dependent increase in serum levels of cytokines.

### IX. Administration of 3,3'-diindolylmethane and substituted 3,3'-diindolylmethane renders BALB/c mice resistant against Y. enterocolitica infection.

Yersinia enterocolitica is a gram-negative, predominantly extracellularly located pathogen which causes enteritis and enterocolitisin humans and rodents. Moreover, systemic infection including abscesses and granulomatous lesions in the spleen and liver occur, particularly in immunocompromised individuals. As in infections with intracellular pathogens, T cells, particularly CD4+ Thl cells, in cooperation with activated macrophages are required for clearance of primary Yersinia infection. The protective host response to yersiniosis is mediated by various proinflammatory cytokines. Neutralization of TNFa, IFNγ, or IL-12 abrogates resistance against this pathogen. Previous studies showed that C57BL/6 mice are resistant against Y. enterocolitica while BALB/c mice are susceptible. Administration of IFN-g, IL-12, or anti-IL-4 antibodies rendered BALB/c mice resistant to yersiniae. As reported above, we have found that DIM can effect an increase in a number of immune response activating cytokines, including IFN-g, IL-12. Here we show that DIM can reduce the severity of yersiniae infection in BALB/c mice.

Female, 6- to 8-week-old BALB/c mice are purchased from Charles River and kept under specific-pathogen-free conditions (positive-pressure cabinet).

Mice are given 30 µg/kg/day of a DIM or one of 24 substituted DIM compounds (Table 1, supra) orally, starting at 1 day prior to infection.

Freshly thawed, plasmid-harboring Y. enterocolitica WA-314 serotype O:8 organisms suspended in 0.1 ml of sterile phosphate-buffered saline (PBS), pH 7.4, are used for intravenous and oral infection as described previously (Autenrieth, et al. 1994.Infect. Immun. 62:2590-2599). The actual number of bacteria administered is determined by plating serial dilutions of the inoculum on Mueller-Hinton agar and counting CFU after an incubation period of 36 h at 26C. For kinetic studies, five mice per group are killed by carbon dioxide asphyxiation on days 1, 3, and 7 postinfection (p.i.) with 5 x 10³ bacteria. Spleens are aseptically removed, and single-cell suspensions prepared by using 5 ml of PBS containing 0.1% bovine serum albumin. Duplicates of 0.1 ml of serial dilutions of these preparations are plated on Mueller-Hinton agar. The limit of detectable CFU is 25 (log₁₀25 = 1.4).

In single end-point studies, bacterial numbers in spleens of treated BALB/c mice 7 days p.i. with 2 x 10³ Y. enterocolitica show significant reductions compared with untreated controls. Consistently, kinetic studies demonstrate time-dependent decreases in spleen bacterial numbers in each of the 25 treatment groups as compared with untreated control mice.

### References

1. Grose, K.R., and Bjeldanes, L.F. Oligomerization of indole-3-carbinol in aqueous acid. Chem. Res. Toxicol., 5: 188-93, 1992.
2. Wattenberg, L.W., and Loub, W.D. Inhibition of polycyclic aromatic hydrocarbon-induced neoplasia by naturally occurring indoles. Cancer Res., 38: 1410-3, 1978.
3. Shertzer, H.G. Protection by indole-3-carbinol against covalent binding of benzo[a]pyrene metabolites to mouse liver DNA and protein. Food Chem. Toxicol., 21: 31-5, 1983.
4. Shertzer, H.G. Indole-3-carbinol protects against covalent binding of benzo[a]pyrene and N-nitrosodimethylamine metabolites to mouse liver macromolecules. Chem. Biol. Interact., 48: 81-90, 1984.
5. Grubbs, C.J., Stele, V.E., Casebolt, T., Juliana, M.M., Eto, I., Whitaker, L.M., Dragnew, K.H., Kellof, G.J., and Lubet, R.L. Chemoprevention of chemically-induced mammary carcinogenesis by indole-3-carbinol. Anticancer Res., 15: 709-16, 1995.
6. Chen, I., McDougal, A., Wang, F., and Safe, S. Aryl hydrocarbon receptor-mediated antiestrogenic and antitumorigenic activity of diindolylmethane. Carcinogenesis,19: 1631-9, 1998.
7. Riby, J.E., Chang, G.H., Firestone, G.L., and Bjeldanes, L.F. Ligand-independent activation of estrogen receptor function by 3, 3'-diindolylmethane in human breast cancer cells. Biochem. Pharmacol., 60: 167-77, 2000.
8. Hong, C., Kim, H.A., Firestone, G.L., and Bjeldanes, L.F. 3,3'-Diindolylmethane (DIM) induces a G(1) cell cycle arrest in human breast cancer cells that is accompanied by Sp1-mediated activation of p21(WAF1/CIP1) expression. Carcinogenesis, 23: 1297-305, 2002.
9. Boehm, U., Klamp, T., Groot, M., and Howard, J.C. Cellular responses to interferon-gamma. Annu. Rev. Immunol., 15: 749-95, 1997.
10. Ikeda, H., Old, L.J., and Schreiber, R.D. The roles of IFN gamma in protection against tumor development and cancer immunoediting. Cytokine Growth Factor Rev., 13: 95-109, 2002.
11. Wimer, B.M. Implications of the analogy between recombinant cytokine toxicities and manifestations of hantavirus infections. Cancer Biother. Radiopharm., 13: 193-207, 1998.
12. Floyd-Smith, G., Wang. Q., and Sen, G.C. Transcriptional induction of the p69 isoform of 2',5'-oligoadenylate synthetase by interferon-beta and interferon-gamma involves three regulatory elements and interferon-stimulated gene factor 3. Exp. Cell Res., 246: 138-47, 1999.
13. Contursi, C., Wang, I.M., Gabriele, L., Gadina, M., O'Shea, J., Morse, H.C. 3rd, and Ozato, K. IFN consensus sequence binding protein potentiates STAT1-dependent activation of IFNgamma-responsive promoters in macrophages. Proc. Natl. Acad. Sci. USA, 97: 91-6, 2000.
14. Schroder, K., Hertzog, P.J., Ravasi, T., and Hume, D.A. Interferon-gamma: an overview of signals, mechanisms and functions. J. Leukoc. Biol., 75: 163-89, 2004.
15. Morinobu, et al. STAT4 serine phosphorylation is critical for IL-12-induced IFN-gamma production but not for cell proliferation. Proc. Natl. Acad. Sci. USA, 99: 12281-6, 2002.
16. Ye, J., Cippitelli, M., Dorman, L., Ortaldo, J.R., Young, H.A. The nuclear factor YY1 suppresses the human gamma interferon promoter through two mechanisms: inhibition of AP1 binding and activation of a silencer element. Mol. Cell Biol.16, 4744-53, 1996.
17. Ghosh, A., Sarkar, S.N., Rowe, T.M., and Sen, G.C. A specific isozyme of 2'-5' oligoadenylate synthetase is a dual function proapoptotic protein of the Bc1-2 family. J. Biol. Chem., 276: 25447-55, 2001.
18. Widschwendter, M., Daxenbichler, G., Dapunt, O., and Marth, C. Effects ofretinoic acid and gamma-interferon on expression of retinoic acid receptor and cellular retinoic acid-binding protein in breast cancer cells. Cancer Res., 55: 2135-9, 1995.
19. Kolla, V., Lindner, D.J., Xiao, W., Borden, E.C., and Kalvakolanu, D.V. Modulation of interferon (IFN)-inducible gene expression by retinoic acid. Up-regulation of STAT1 protein in IFN-unresponsive cells. J. Biol. Chem., 271: 10508-14, 1996.
20. Lindner, D.J., Kolla, V., Kalvakolanu, D.V., and Borden, E.C. Tamoxifen enhances interferon-regulated gene expression in breast cancer cells. Mol. Cell. Biochem., 167: 169-77, 1997.
21. Marincola, F.M., Jaffee, E.M., Hicklin, D.J., and Ferrone, S. Escape of human solid tumors from T-cell recognition: molecular mechanisms and functional significance. Adv. Immunol., 74: 181-273, 2000.
22. Goodenow, R.S., Vogel, J.M., and Linsk, R.L. Histocompatibility antigens on murine tumors. Science, 230: 777-83, 1985.
23. Tanaka, et al. Role of the major histocompatibility complex class I antigens in tumor growth and metastasis. Annu. Rev. Immunol., 6: 359-80, 1988.
24. David-Watine, B., Israel, A., and Kourilsky, P. The regulation and expression of MHC class I genes. Immunol. Today, 11: 286-92, 1990.
25. Ohtsukasa, S., Okabe, S., Yamashita, H., Iwai, T., and Sugihara, K. Increased expression of CEA and MHC class I in colorectal cancer cell lines exposed to chemotherapy drugs. J. Cancer Res. Clin. Oncol., 129: 719-26, 2003.
26. Geissmann, F., Revy, P., Brousse, N., Lepelletier, Y., Folli, C., Durandy, A., Chambon, P., and Dy, M. Retinoids regulate survival and antigen presentation by immature dendritic cells. J. Exp. Med., 198: 623-34, 2003.
27. Chatterji, U., Riby, J.E., Taniguchi, T., Bjeldanes, E.L., Bjeldanes, L.F., and Firestone, G.L. Indole-3-carbinol stimulates transcription of the interferon gamma receptor 1 gene and augments interferon responsiveness in human breast cancer cells. Carcinogenesis, 25:1119-28,2004
28. Staub, R.E., Feng, C., Onisko, B., Bailey, G.S., Firestone, G.L., and Bjeldanes, L.F. Fate of indole-3-carbinol in cultured human breast tumor cells. Chem. Res. Toxicol., 15: 101-9, 2002.
29. Wiatrak, B.J. Overview of recurrent respiratory papillomatosis. Curr. Opin. Otolaryngol. Head Neck Surg., 11: 433-41, 2003.
30. Aubom, K.J. Therapy for recurrent respiratory papillomatosis. Antivir. Ther., 7:1-9, 2002.
31. Coll D.A., Rosen, C.A., Aubom, K., Potsic, W.P., and Bradlow, H.L. Treatment of recurrent respiratory papillomatosis with indole-3-carbinol Am. J. Otolaryngol., 18: 283-5, 1997.
32. Rosen, C.A., Woodson, G.E., Thompson, J.W., Hengesteg, A.P., and Bradlow, H.L. Preliminary results of the use of indole-3-carbinol for recurrent respiratory papillomatosis Otolaryngol. Head Neck Surg., 118: 810-5, 1998.
33. Gissmann, L., Diehl, V., Schultz-Coulon, H.J., and zur Hausen, H. Molecular cloning and characterization of human papilloma virus DNA derived from a laryngeal papilloma. J. Virol., 44: 393-400, 1982.
34. Kashima, H.K., Mounts, P., and Shah, K. Recurrent respiratory papillomatosis. Obstet. Gynecol. Clin. North Am., 23: 699-706, 1996.
35. Yuan, F., Chen, D.Z., Liu, K., Sepkovic, D.W., Bradlow, H.L., and Aubom, K. Antiestrogenic activities of indole-3-carbinol in cervical cells: implication for prevention of cervical cancer. Anticancer Res. 19: 1673-80, 1999.
36. Cover, C.M., Hsieh, S.J., Tran, S.H., Hallden, G., Kim, G.S., Bjeldanes, L.F., and Firestone, G.L. Indole-3-carbinol inhibits the expression of cyclin-dependent kinase-6 and induces a G1 cell cycle arrest of human breast cancer cells independent of estrogen receptor signaling. J. Biol. Chem., 273: 3838-47, 1998.
37. Hong, C., Firestone, G.L., and Bjeldanes, L.F. Bcl-2 family-mediated apoptotic effects of 3,3'-diindolylmethane (DIM) in human breast cancer cells. Biochem. Pharmacol., 63: 1085-97,2002.

The foregoing descriptions of particular embodiments and examples are offered by way of illustration and not by way of limitation. All publications and patent applications cited in this specification and all references cited therein are herein incorporated by reference as if each individual publication or patent application or reference were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

For the avoidance of doubt, the present application relates to the subject-matter defined in the following numbered paragraphs (referred to as "para" or "paras").
1. A method of providing an immune response activator to a patient determined to be in need thereof, comprising the steps of:
   administering to the patient a predetermined amount of an immune response activating, optionally substituted DIM; and
   detecting a resultant immune response activation in the patient.
2. The method of para 1 wherein the immune response activation is and is detected as an increase in T-cell proliferation, cytokine production, cytokine receptor expression, or cytokine signaling.
3. The method of para 1 wherein the cytokine is selected from the group consisting of IL-6, G-CSF, IL-12, TNF-α and IFNγ
4. The method of para 1 wherein the administering step is performed by oral or intravenous administration.
5. The method of para 1 wherein the patient is determined to be immune-compromised, or subject to an infection or a neoplasia.
6. The method of para 1 wherein the method further comprises, prior to the contacting step, determining that the host is in need of the immune response activator
7. The method of para 1 wherein the optionally substituted 3,3'-diindolylmethane has the formula: 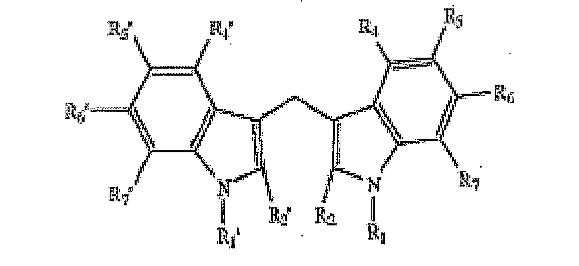 where Rub.1, R.sub.2, R.sub.4, R.sub.5, R.sub.6, R.sub.7, R.sub.1', R.sub.2', R.sub.4', R.sub.5', R.sub.6' and R.sub.7' individually and independently, are hydrogen or a substituent selected from the group consisting of a halogen, a hydroxyl, a linear or branched alkyl or alkoxy group of one to ten carbons, and a nitro group.
8. The method of para 7 wherein R.sub.1, R.sub.2, R.sub.4, R.sub.5, R.sub.6, R.sub.7, R.sub.1', R.sub.2', R.sub.4', R.sub.5', R.sub.6' and R.sub.7' include a substituent selected from the group consisting of a halogen, a hydroxyl, a linear or branched alkyl or alkoxy group of one to ten carbons, and a nitro group.
9. The method of para 8 wherein the linear or branched alkyl or alkoxy group is one to five carbons.
10. The method of para 8 wherein the halogen is selected from the group consisting of chlorine, bromine and fluorine.
11. The method of para 8, wherein R.sub.1, R.sub.2, R.sub.4, R.sub.6, R.sub.7, R.sub.1', R.sub.2', R.sub.4', R.sub.6', and R.sub.7' are hydrogen, and R.sub.5 and R.sub.5' are a halogen.
12. The method of para 8, wherein R.sub.2, R.sub.4, R.sub.5, R.sub.6, R.sub.7, R.sub. 2', R.sub.4', R.sub.5', R.sub.6', and R.sub.7' are hydrogen, and R.sub.1 and R.sub.1' are an alkyl or alkoxyl having from one to ten carbons.
13. The method of para 8, wherein Rub.1, R.sub.4, R.sub.5, R.sub.6, R.sub.7, R.sub.1', R.sub.4', R.sub.5', R.sub.6', and R.sub.7' are hydrogen, and R.sub.2 and R.sub.2' are an alkyl of one to ten carbons.
14. The method of para 8, wherein R.sub.1, R.sub.2, R.sub.4, R.sub.6, R.sub.7, R.sub.1', R.sub.2', R.sub.4', R.sub.6', and R.sub.7' are hydrogen, and R.sub.5 and R.sub.5' are nitro.
15. The method of paras 1, 3, 4, 5 or 6 wherein the optionally substituted 3,3'-diindolylmethane is 3,3'-diindolylmethane.
16. The method of paras 1, 3, 4, 5 or 6 wherein the optionally substituted 3,3'-diindolylmethane is perfluoro-3,3'-diindolylmethane.
17. A pharmaceutical composition comprising predetermined amounts of an immune response activating, optionally substituted DIM, and a cytokine.
18. A pharmaceutical composition comprising predetermined amounts of an immune response activating, optionally substituted DIM, and a cytokine selected from the group consisting of: epidermal growth factor (EGF), platelet-derived growth factor (PDGF), fibroblast growth factors (FGFs), transforming growth factors-β (TGFs-β), transforming growth factor-α (TGF-α), erythropoietin (Epo), insulin-like growth factor-I (IGF-I), insulin-like growth factor-II (IGF-II), interleukin-1 (IL-1), interleukin-2 (IL-2), interleukin-6 (IL-6), interleukin-8 (IL-8), tumor necrosis factor-α(TNF-α), tumor necrosis factor-β (TNF-β), interferon-γ (INF-γ), and colony stimulating factors (CSFs).

## Claims

1. An immune response activating, optionally substituted 3, 3'-diindolylmethane for use as an immune activator.

2. An immune response activating, optionally substituted 3, 3'-diindolylmethane according to claim 1, wherein the immune response activating, optionally substituted 3, 3'-diindolylmethane is for treating an immune-compromised patient.

3. An immune response activating, optionally substituted 3, 3'-diindolylmethane according to claim 1 or claim 2, wherein the optionally substituted 3,3'-diindolymethane has the formula: 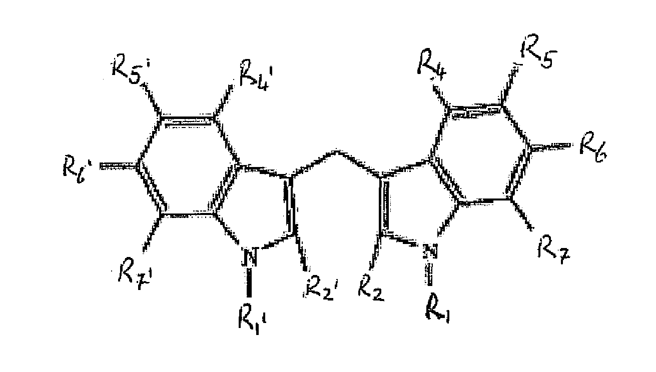 where R₁, R₂, R₄, R₅, R₆, R₇, R_{1'}, R_{2'}, R_{4'}, R_{5'}, R_{6'} and R_{7'} individually and independently, are hydrogen or a substituent selected from the group consisting of a halogen, a hydroxyl, a linear or branched alkyl or alkoxy group of one to ten carbons, and a nitro group.

4. The immune response activating, optionally substituted 3, 3'-diindolylmethane according to claim 3 wherein at least one of R₁, R₂, R₄, R₅, R₆, R₇, R_{1'}, R_{2'}, R_{4'}, R_{5'}, R_{6'} and R₇, is a substituent selected from the group consisting of a halogen, a hydroxyl, a linear or branched alkyl or alkoxy group of one to ten carbons, and a nitro group.

5. The immune response activating, optionally substituted 3, 3'-diindalylmethane according to claim 4 wherein R₁, R₂, R₄, R₆, R₇, R_{1',} R_{2'}, R_{4'}, R_{6'} and R_{7'} are hydrogen and R₅ and R_{5'} are a halogen.

6. The immune response activating, optionally substituted 3, 3'-diindolylmethane according to claim 1 wherein the optionally substituted 3,3'-diindolymethane is selected from the group consisting of
5,5'-dichlorodiindolylmethane; 5,5'-dibromo-diindolylmethane;
5, 5'-difluorodiindolylmethane; perfluoro-3,3'-diindolylmethane;
5,5'-dimethyldiindolylmethane; 5,5'-diethyl-diindolylmethane;
5,5'-dipropyldiindolylmethane; 5,5'-dibutyl-diindolylmethane;
5,5'-dipentyldiindolylmethane; 5,5'-dimethoxy-diindolylmethane;
5,5'-diethoxydiindolylmethane; 5,5'-dipropyloxy-diindolylmethane;
5,5'-dibutyloxydiindolylmethane; 5,5'-diamyloxy-diindolylmethane;
N,N'-dimethyldiindolylmethane; N,N'-diethyl-diindolylmethane;
N,N'-dipropyldiindolylmethane; N,N'-dibutyl-diindolylmethane;
N,N'-dipentyldiindolylmethane; 2,2-dimethyl-diindolylmethane;
2,2'-diethyldiindolylmethane; 2,2'-dipropyl-diindolylmethane;
2,2'-dibutyldiindolylmethane; and 2,2'-dipentyl-diindolylmethane.

7. An immune response activating, optionally substituted 3, 3'-diindolylmethane according to any one of claims 1 to 6, wherein the immune response activating, optionally substituted 3, 3'-diindolylmethane is for use in a method comprising the steps of (a) administering to the patient a predetermined amount of an immune response activating, optionally substituted DIM; and (b) detecting a resultant immune response activation in the patient.

8. The immune response activating, optionally substituted 3, 3'-diindolylmethane according to claim 1 wherein the use is in a host that is an animal or a human.

9. The immune response activating, optionally substituted 3, 3'-diindolylmethane according to any one of the preceding claims, wherein the optionally substituted 3, 3'-diindolylmethane is for use in a human patient determined to be subject or predisposed to a pathology which can be ameliorated with an increased immune response, and wherein the resultant immune response activation is manifested as a reduction in the pathology or progress of the pathology, wherein the pathology is selected from the group consisting of viral, bacterial and fungal infections.

10. The immune response activating, optionally substituted 3, 3'-diindolylmethane according to claim 1, wherein the use of the immune response activating, optionally substituted 3, 3'-diindolylmethane is with one or more therapeutic agents.

11. A pharmaceutical composition comprising predetermined amounts of an immune response activating, optionally substituted DIM, and a cytokine.

12. The pharmaceutical composition according to claim 11, wherein the composition is for treating an immune-compromised patient.

13. The pharmaceutical composition according to claim 11 or claim 12, wherein the cytokine is selected from the group consisting of: selected from the group consisting of: interferon-γ (INF-γ), epidermal growth factor (EGF), platelet-derived growth factor (PDGF), fibroblast growth factors (FGFs), transforming growth factors-β (TGFs-β), transforming growth factor-α (TGF-α), erythropoietin (Epo), insulin-like growth factor-I (IGF-I), insulin- like growth factor-II (IGF-II), interleukin-1 (IL-I), interleukin-2 (IL-2), interleukin-6 (IL-6), interleukin-8 (IL-8), tumor necrosis factor-α (TNF-α), tumor necrosis factor-β (TNF-β), and colony stimulating factors (CSFs)

14. The pharmaceutical composition according to any one of claims 11 to 13, wherein the optionally substituted 3,3'-diindolymethane has the formula: 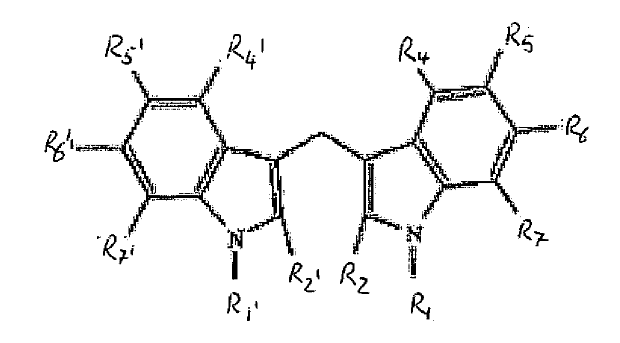 where R₁, R₂, R₄, R₅, R₆, R₇, R_{1'}, R_{2'}, R_{4'}, R_{5'}, R_{6'} and R_{7'} individually and independently, are hydrogen or a substituent selected from the group consisting of a halogen, a hydroxyl, a linear or branched alkyl or alkoxy group of one to ten carbons, and a nitro group.

15. The pharmaceutical composition according to claim 14, wherein the optionally substituted 3,3'-diindolymethane is as defined in any one of claims 4 to 6.
